# EUROPEAN PATENT APPLICATION

(11) **EP 3 744 349 A1**
(43) Date of publication of application: **02.12.2020**
(21) Application number: 19740897.4
(22) Date of filing: 22.01.2019
(51) Int. Cl.: A61K 47/32, A61K 9/70, A61K 47/10

(54) **ADHESIVE SHEET FOR ATTACHMENT TO SKIN**

(30) Priority: 22.01.2018 JP 2018008106
(71) Applicant: Kaneka Corporation, Osaka-shi, Osaka 530-8288 (JP)
(72) Inventor: OGINO Hiroyuki, Takasago-shi, Hyogo 676-8688 (JP); AKAZAWA Mitsuji, Takasago-shi, Hyogo 676-8688 (JP); GOTOU Masaoki, Takasago-shi, Hyogo 676-8688 (JP); MIZUTANI Masaya, Takasago-shi, Hyogo 676-8688 (JP); TANAKA Tatsuyoshi, Takasago-shi, Hyogo 676-8688 (JP)
(74) Representative: Hoffmann Eitle
(86) International application number: PCT/JP2019/001792
(87) International publication number: WO 2019/142940

(57) **Abstract**

It is an object of the present invention to provide an adhesive sheet for skin patch, having adhesive force and cohesive force that are practicable as a pharmaceutical product and exhibiting high drug solubility and sufficient drug skin permeability. According to the present invention, provided is an adhesive sheet for skin patch, having an adhesive layer on a support, wherein the adhesive layer comprises, at least, a thermoplastic elastomer and a long-chain branched alcohol having a total carbon number of 13 or more, the long-chain branched alcohol is comprised in an amount of 40 parts by weight or more and 200 parts by weight or less, with respect to 100 parts by weight of the thermoplastic elastomer, and the adhesive layer does not comprise a tackifier, or comprises a tackifier in an amount of 30% by weight or less, with respect to the amount of the entire adhesive layer.

## Description

### Technical Field

The present invention relates to an adhesive sheet for skin patch, which is composed of a thermoplastic elastomer and a long-chain branched alcohol. More specifically, the present invention relates to an adhesive sheet for skin patch, which exhibits high drug solubility and sufficient drug skin permeability, while having sufficient adhesiveness to the skin.

### Background Art

As an adhesive layer used in an adhesive sheet for skin patch, a rubber-based, acrylic, or silicone-based lipophilic adhesive base has been commonly used. Among others, the rubber-based adhesive base has been broadly used, since additives are easily mixed into the rubber-based adhesive base, in comparison to other adhesive bases. When such a rubber-based adhesive base is used, it is considered that a tackifier is essentially added to the adhesive base in order to provide adhesiveness. However, it has been revealed that skin irritation is caused by such a tackifier.

On the other hand, when an adhesive sheet for skin patch is allowed to comprise a drug, so that it is used as a patch, it is essential for the patch to have high skin permeability, in order to obtain sufficient medicinal effects. For having high skin permeability, the adhesive layer must contain a high concentration of drug therein. If such a high concentration of drug is added into a rubber-based adhesive base, the drug is precipitated from the adhesive base as a result of crystallization of the drug. Thus, a method of dissolving a drug in a dissolving agent and then adding it into an adhesive base is commonly applied. However, this method is problematic in that, if a large amount of dissolving agent is used, the cohesive force of an adhesive base decreases and thereby, the obtained mixture cannot serve as an adhesive base.

There has been proposed a patch, in which suppression of the precipitation of drug crystals and the improvement of the drug release rate can be achieved by using 2-octyl-1-dodecanol or isostearyl alcohol as a dissolving agent (Patent Document 1). However, since the drug (lidocaine) is used in a low concentration, it is anticipated that crystal precipitation of the drug can be easily suppressed, and further that the skin permeability of the drug will not be high.

Moreover, a rubber-based adhesive base comprising lauryl alcohol or oleyl alcohol, instead of a plasticizer, and a large amount of tackifier, has been proposed (Patent Document 2). However, as a result of the studies conducted by the present inventors, it has been revealed that the cohesive force of the adhesive base described in Patent Document 2 is low, and that a drug is not dissolved therein. Furthermore, it has also been revealed that since this rubber-based adhesive base comprises a large amount of tackifier, it causes strong skin irritation when it is peeled from the skin.

### Prior Art Documents

### Patent Documents

Patent Document 1: Japanese Patent Publication (Kokai) No. 2008-266198 A
Patent Document 2: Japanese Patent Publication (Kokai) No. 2003-063955 A

### Summary of Invention

### Object to be Solved by the Invention

It is an object of the present invention to provide an adhesive sheet for skin patch, having adhesive force and cohesive force that are practicable as a pharmaceutical product and exhibiting high drug solubility and sufficient drug skin permeability.

### Means for Solving the Object

The present inventors have conducted intensive studies directed towards achieving the aforementioned object. As a result, the present inventors have found that an adhesive sheet for skin patch, having adhesive force and cohesive force that are practicable as a pharmaceutical product and exhibiting high drug solubility and sufficient drug skin permeability, can be provided by adopting the configuration of an adhesive sheet for skin patch, having an adhesive layer on a support, wherein the adhesive layer comprises, at least, a thermoplastic elastomer and a long-chain branched alcohol having a total carbon number of 13 or more, the long-chain branched alcohol is comprised in an amount of 40 parts by weight or more and 200 parts by weight or less, with respect to 100 parts by weight of the thermoplastic elastomer, and the adhesive layer does not comprise a tackifier, or comprises a tackifier in an amount of 30% by weight or less, with respect to the amount of the entire adhesive layer, thereby completing the present invention.

Specifically, the present invention relates to the following [1] to [17].
[1] An adhesive sheet for skin patch, having an adhesive layer on a support, wherein
   the adhesive layer comprises, at least, a thermoplastic elastomer and a long-chain branched alcohol having a total carbon number of 13 or more,
   the long-chain branched alcohol is comprised in an amount of 40 parts by weight or more and 200 parts by weight or less, with respect to 100 parts by weight of the thermoplastic elastomer, and
   the adhesive layer does not comprise a tackifier, or comprises a tackifier in an amount of 30% by weight or less, with respect to the amount of the entire adhesive layer.
[2] The adhesive sheet for skin patch according to [1], which comprises the long-chain branched alcohol in an amount of 50 parts by weight or more and 200 parts by weight or less, with respect to 100 parts by weight of the thermoplastic elastomer.
[3] The adhesive sheet for skin patch according to [1] or [2], which comprises the long-chain branched alcohol in an amount of 10% by weight or more, with respect to the amount of the entire adhesive layer.
[4] The adhesive sheet for skin patch according to [1] to [3], which comprises the long-chain branched alcohol in an amount of 20% by weight or more, with respect to the amount of the entire adhesive layer.
[5] The adhesive sheet for skin patch according to any one of [1] to [4], wherein the thermoplastic elastomer is a styrene-based block copolymer.
[6] The adhesive sheet for skin patch according to [5], wherein the styrene-based block copolymer is a mixture consisting of a styrene-isoprene-styrene block copolymer and a styrene-isoprene block copolymer.
[7] The adhesive sheet for skin patch according to [6], wherein the content of the styrene-isoprene block copolymer in the mixture is 30% by weight or more.
[8] The adhesive sheet for skin patch according to [6] or [7], wherein the content of the styrene-isoprene block copolymer in the mixture is 50% by weight or more.
[9] The adhesive sheet for skin patch according to any one of [1] to [8], wherein the content of the tackifier is 20% by weight or less, with respect to the content of the entire adhesive layer.
[10] The adhesive sheet for skin patch according to any one of [1] to [9], wherein the content of the tackifier is 15% by weight or less, with respect to the content of the entire adhesive layer.
[11] The adhesive sheet for skin patch according to any one of [1] to [10], wherein the content of the tackifier is 10% by weight or less, with respect to the content of the entire adhesive layer.
[12] The adhesive sheet for skin patch according to any one of [1] to [11], which does not comprise a tackifier.
[13] The adhesive sheet for skin patch according to any one of [1] to [12], wherein the adhesive layer further comprises an alcohol solvent in an amount of 40 parts by weight or less, with respect to 100 parts by weight of the long-chain branched alcohol.
[14] The adhesive sheet for skin patch according to [13], wherein the alcohol solvent is propylene glycol mono-fatty acid ester.
[15] An adhesive sheet for skin patch, having an adhesive layer on a support, wherein the adhesive layer comprises, at least, a thermoplastic elastomer and, as a plasticizer, a long-chain branched alcohol having a total carbon number of, at least, 13 or more, and the plasticizer is comprised in an amount of 20% by weight or more, with respect to the amount of the entire adhesive layer.
[16] The adhesive sheet for skin patch according to any one of [1] to [15], wherein the adhesive layer further comprises a drug or a pharmaceutically acceptable salt thereof.
[17] The adhesive sheet for skin patch according to [16], which is used as a patch.

### Advantageous Effects of Invention

According to the present invention, an adhesive sheet for skin patch, having adhesive force and cohesive force that are practicable as a pharmaceutical product and exhibiting high drug solubility and sufficient drug skin permeability, can be provided.

### Brief Description of Drawings

[Fig.1] Fig.1 shows the results obtained by measuring a lidocaine-containing local anesthetic preparation comprising an existing rubber-based adhesive base and the patch of Example 6, in terms of the accumulated amount of the drug permeating through the skin.
[Fig.2] Fig.2 shows the results obtained by measuring an existing 5% lidocaine-containing hydrogel patch and the patches of Example 6 and Example 7, in terms of the accumulated amount of the drug permeating through the skin.
[Fig.3] Fig.3 shows the results obtained by measuring an existing 4% tetracaine-containing gel and the patches of Example 11 and Example 12, in terms of the accumulated amount of the drug permeating through the skin.

### Embodiment of Carrying out the Invention

The embodiments of the present invention will be described below.

The present invention relates to an adhesive sheet for skin patch, having an adhesive layer on a support, wherein
the adhesive layer comprises, at least, a thermoplastic elastomer and a long-chain branched alcohol having a total carbon number of 13 or more,
the long-chain branched alcohol is comprised in an amount of 40 parts by weight or more and 200 parts by weight or less, with respect to 100 parts by weight of the thermoplastic elastomer, and
the adhesive layer does not comprise a tackifier, or comprises a tackifier in an amount of 30% by weight or less, with respect to the amount of the entire adhesive layer.

### < Concerning components constituting adhesive sheet for skin patch >

The adhesive layer of the present invention comprises, at least, (a) a thermoplastic elastomer and (b) a long-chain branched alcohol having a total carbon number of 13 or more.

### (a) Thermoplastic elastomer

The "thermoplastic elastomer" used in the present invention is an elastomer exhibiting thermoplasticity, which is softened by heating to exhibit liquidity and is returned to a rubber-like elastic body by cooling. Examples of such a thermoplastic elastomer may include various types of thermoplastic elastomers, such as urethane-based, acrylic, styrene-based, or olefinic thermoplastic elastomers. In particular, from the viewpoint of achieving both sufficient skin adhesiveness and low skin irritation, which is the object of the present invention, a styrene-based thermoplastic elastomer, in particular, a styrene-based block copolymer is preferably used.

Specific examples of the styrene-based block copolymer used as a thermoplastic elastomer may include a styrene-butadiene block copolymer, a styrene-butadiene-styrene block copolymer, a styrene-isoprene block copolymer, a styrene-isoprene-styrene block copolymer, a styrene-ethylene/butylene block copolymer, a styrene-ethylene/butylene-styrene block copolymer, a styrene-ethylene/propylene block copolymer, a styrene-ethylene/propylene-styrene block copolymer, a styrene-isobutylene block copolymer, and a styrene-isobutylene-styrene block copolymer. Besides, in the above description, the term "ethylene/butylene" means a copolymeric block of ethylene and butylene, whereas the term "ethylene/propylene" means a copolymeric block of ethylene and propylene. These styrene-based block copolymers may be used alone as a single type, or in combination of two or more types.

Among the above-described styrene-based block copolymers, one or two or more types selected from the group consisting of styrene-isoprene-styrene block copolymers and styrene-isoprene block copolymers are particularly preferably used, from the viewpoint of the achievement of both sufficient skin adhesiveness and suppression of adhesive residue by the improvement of the cohesive force of the adhesive layer, and also, the availability and handing ability of products for patches. As such a styrene-based block copolymer, a mixture of a styrene-isoprene-styrene block copolymer and a styrene-isoprene block copolymer is most preferable.

When a mixture of a styrene-isoprene-styrene block copolymer and a styrene-isoprene block copolymer is used as a styrene-based block copolymer, the content of the styrene-isoprene block copolymer in the mixture is preferably 15% by weight or more, more preferably 20% by weight or more, even more preferably 30% by weight or more, further preferably 40% by weight or more, and particularly preferably 50% by weight or more. The upper limit of the content of the styrene-isoprene block copolymer in the mixture is preferably 80% by weight or less.

For the purpose of the present invention, a styrene-isoprene-styrene block copolymer, in which the content of styrene in the copolymer is 5% by weight or more and 60% by weight or less, is preferable; and a styrene-isoprene-styrene block copolymer, in which the content of styrene in the copolymer is 10% by weight or more and 50% by weight or less, is more preferable. Moreover, a styrene-isoprene-styrene block copolymer having a weight average molecular weight measured by gel permeation chromatography (GPC) of 20,000 or more and 500,000 or less is preferable; and a styrene-isoprene-styrene block copolymer having a weight average molecular weight measured by gel permeation chromatography (GPC) of 30,000 or more and 300,000 or less is more preferable. Furthermore, a styrene-isoprene block copolymer, in which the content of styrene in the copolymer is 5% by weight or more and 50% by weight or less, is preferable; and a styrene-isoprene block copolymer, in which the content of styrene in the copolymer is 10% by weight or more and 40% by weight or less, is more preferable. Further, a styrene-isoprene block copolymer having a weight average molecular weight measured by gel permeation chromatography (GPC) of 10,000 or more and 500,000 or less is preferable; and a styrene-isoprene block copolymer having a weight average molecular weight measured by gel permeation chromatography (GPC) of 20,000 or more and 300,000 or less is more preferable.

As such a styrene-isoprene-styrene block copolymer and a styrene-isoprene block copolymer, copolymers produced by a known method can be used. In addition, as such a styrene-isoprene-styrene block copolymer and a styrene-isoprene block copolymer, commercially available products that satisfy the above-described properties can also be used. Also, a mixture of a styrene-isoprene-styrene block copolymer and a styrene-isoprene block copolymer is commercially available. A commercially available product that is a mixture obtained by mixing, at the above-described mixing ratio, a styrene-isoprene-styrene block copolymer and a styrene-isoprene block copolymer, which satisfy the above-described properties, can be preferably used.

Examples of the commercially available product may include: "KRATON (registered trademark) D1111," "KRATON (registered trademark) D1163," "KRATON (registered trademark) D1113" and "KRATON (registered trademark) D1119" manufactured by KRATON POLYMERS; "JSR SIS (registered trademark) 5229," "JSR SIS (registered trademark) 5002," "JSR SIS (registered trademark) 5403" and "JSR SIS (registered trademark) 5505," manufactured by JSR; and "Quintac (registered trademark) 3421," "Quintac (registered trademark) 3433N," "Quintac (registered trademark) 3520," "Quintac (registered trademark) 3450," and "Quintac3270" manufactured by ZEON CORPORATION. Among these, from the viewpoint of the mixing ratio between the above-described triblock copolymer and diblock copolymer and the viscosity of a solution, "KRATON (registered trademark) D1163," "KRATON (registered trademark) D1113," "JSR SIS (registered trademark) 5403," "JSR SIS (registered trademark) 5505," "Quintac (registered trademark) 3433N," and "Quintac (registered trademark) 3520" are preferable; and "JSR SIS (registered trademark) 5505" and "Quintac (registered trademark) 3520" are particularly preferably used. Each of these styrene-based block copolymers is a mixture of a styrene-isoprene-styrene block copolymer and a styrene-isoprene block copolymer, in which the content of the styrene-isoprene block copolymer in the mixture is 50% by weight or more.

If the content of the thermoplastic elastomer in the adhesive layer is too small, it becomes difficult to maintain the shape of the adhesive layer. On the other hand, if the content of the thermoplastic elastomer in the adhesive layer is too large, skin adhesiveness becomes insufficient. Accordingly, the content of the thermoplastic elastomer in the adhesive layer of the adhesive sheet for skin patch of the present invention is preferably 20% by weight or more and 65% by weight or less, more preferably 25% by weight or more and 65% by weight or less, and particularly preferably 30% by weight or more and 60% by weight or less.

### (b) Long-chain branched alcohol having a total carbon number of 13 or more

The "long-chain branched alcohol having a total carbon number of 13 or more" that can be used in the present invention is an aliphatic alcohol having a branch structure and also having a total carbon number of 13 or more. It was found that an alcohol having a branch structure and also having a total carbon number of 13 or more has plasticizing effect, and thus that such an alcohol can serve as an adhesive layer by being combined with a styrene-isoprene-styrene block copolymer, although a plasticizer such as liquid paraffin as a typical example is not added thereto. In the case of an alcohol having a total carbon number of 12 or less, it has low plasticizing effect, and the cohesive force of the adhesive is significantly reduced. Hence, even when such an alcohol having a total carbon number of 12 or less is added in a small amount, it cannot serve as an adhesive. On the other hand, in the case of a linear alcohol, it has low plasticizing effect, and also, when such a linear alcohol is used as an adhesive, the component bleeds out.

The content of the long-chain branched alcohol is preferably 10% by weight or more, more preferably 20% by weight or more, further preferably 30% by weight or more, and particularly preferably 40% by weight or more, based on the total content of the adhesive layer. The upper limit of the content of the long-chain branched alcohol is 75% by weight or less based on the total content of the adhesive layer.

The lower limit of the content of the long-chain branched alcohol in the thermoplastic elastomer is 40 parts by weight or more, preferably 50 parts by weight or more, more preferably 75 parts by weight or more, and further preferably 100 parts by weight or more, with respect to 100 parts by weight of the thermoplastic elastomer. The upper limit of the content of the long-chain branched alcohol in the thermoplastic elastomer is 200 parts by weight or less, preferably 175 parts by weight or less, and more preferably 150 parts by weight or less, with respect to 100 parts by weight of the thermoplastic elastomer.

Examples of the long-chain branched alcohol that can be used for the purpose of the present invention may include 2-hexyl-1-decanol, 2-octyl-1-dodecanol, isostearyl alcohol, and 2-decyl-1-tetradecanol. Among these, from the viewpoint of keeping balance between the cohesiveness and adhesiveness of the adhesive layer and the solubility of a drug, 2-hexyl-1-decanol, 2-octyl-1-dodecanol, and 2-decyl-1-tetradecanol are preferable. These long-chain branched alcohols may be used alone as a single type, or in combination of two or more types.

### (c) Tackifier

The adhesive layer of the present invention does not comprise a tackifier, or comprises a tackifier in an amount of 30% by weight or less, with respect to the amount of the entire adhesive layer.

The term "tackifier" used herein means a tackifier that is commonly used in the field of patches. Examples of the tackifier may include a rosin resin, a polyterpene resin, a coumarone-indene resin, a petroleum resin, a terpene resin, a terpene-phenolic resin, and an aliphatic saturated hydrocarbon resin. If a large amount of tackifier is added, the releasability of a drug is reduced, and skin irritation is increased. Accordingly, the content of the tackifier is, with respect to the total amount of the adhesive components, more preferably 25% by weight or less, even more preferably 20% by weight or less, further preferably 15% by weight or less, still further preferably 10% by weight or less, particularly preferably 5% by weight or less, and most preferably, no tackifier is comprised.

### (d) Drug or pharmaceutically acceptable salt thereof

In the present invention, (d) a drug or a pharmaceutically acceptable salt thereof is further added to the adhesive layer of the adhesive sheet for skin patch, so that the present adhesive sheet for skin patch can be used as a patch.

The "drug or a pharmaceutically acceptable salt thereof" of the present invention means a drug or a salt thereof used for transdermal absorption, and is not particularly limited. Examples of the drug may include: anti-inflammatory analgesics, such as acetaminophen, phenacetin, mefenamic acid, diclofenac sodium, flufenamic acid, aspirin, sodium salicylate, methyl salicylate, glycol salicylate, aminopyrine, alclofenac, ibuprofen, naproxen, flurbiprofen, ketoprofen, amfenac sodium, mepirizole, indomethacin, piroxicam, and felbinac; steroidal anti-inflammatory agents, such as hydrocortisone, triamcinolone, dexamethasone, and prednisolone; vasodilators, such as diltiazem hydrochloride, pentaerythritol tetranitrate, isosorbide nitrate, tradipil, nicorandil, nitroglycerol, prenylamine lactate, molsidomine, aluminum nitrite, tolazoline hydrochloride, and nifedipine; antiarrhythmic drugs, such as procaineamide hydrochloride, lidocaine hydrochloride, propranolol hydrochloride, alprenolol hydrochloride, atenolol, nadolol, metoprolol tartrate, ajmaline, disopyramide, and mexiletine hydrochloride; antihypertensive drugs, such as ecarazine hydrochloride, indapamide, clonidine hydrochloride, bunitrolol hydrochloride, labetalol hydrochloride, captopril, guanabenz acetate, mebutamate, and bethanidine sulfate; antitussive expectorants, such as carbetapentane citrate, cloperastine, oxeladin tannate, cloputinol hydrochloride, clofedanol hydrochloride, noscapine hydrochloride, ephedrine hydrochloride, isoproterenol hydrochloride, cloriprenaline hydrochloride, methoxyphenamine hydrochloride, procaterol hydrochloride, tulobuterol hydrochloride, clenputerol hydrochloride, and ketotifen fumarate; antineoplastic drugs, such as cyclophosphamide, fluorouracil, degafur, mitomycin C, procarbazine hydrochloride, doxifluridine, and ranimustine; local anesthetics, such as ethyl aminobenzoate, tetracaine hydrochloride, brocaine hydrochloride, dibucaine hydrochloride, lidocaine hydrochloride, oxybuprocaine hydrochloride, and propitocaine hydrochloride; drugs, such as isoconazole hydrochloride, efinaconazole, itraconazole, neticonazole hydrochloride, oxiconazole hydrochloride, ketoconazole, terbinafine hydrochloride, butenafine hydrochloride, miconazole nitrate, lanoconazole, and luliconazole; hormone agents, such as propylthiouracil, thiamazole, metelonone acetate, estradiol, estriol, and progesterone; antihistamine agents, such as diphenhydramine hydrochloride, chlorpheniramine maleate, promethazine, dyproheptadine hydrochloride, and diphenylpyraline hydrochloride; anticoagulants, such as warfarin potassium and ticlopidine hydrochloride; anticonvulsive agents, such as atropine methylbromide, scopolamine, and baclofen; systemic anesthetics, such as thiopental sodium and pentobarbital sodium; hypnotics and/or analgesics, such as bromovalenylurea, amobarbital, and phenobarbital; antiepileptic drugs such as phenytoin sodium; analeptics and/or stimulant drugs such as methamphetamine hydrochloride; antidizziness drugs, such as difendol hydrochloride and betahistine mesilate; psychoneurotic agents, such as chlorpromazine hydrochloride, thioridazine, meprobamate, imipramine hydrochloride, chlordiazepoxide, diazepam, risperidone, paliperidone, olanzapine, aripiprazole, paroxetine, duloxetine, quetiapine, and tiapride; muscle relaxants, such as suxamethonium hydrochloride and eperisone hydrochloride; autonomic drugs, such as neostigmine bromide and bethanechol chloride; antiparkinson agents, such as amantadine hydrochloride, rotigotine, and ropinirole; anti-Alzheimer's dementia drugs, such as donepezil, galanthamine, memantine, and rivastigmine; diuretics, such as hydroflumethiazide, isosorbide, and furosemide; vasoconstrictors such as phenylephrine hydrochloride; respiratory stimulants, such as lobeline bromide, dimorpholamine, and naloxone hydrochloride; peptic ulcer therapeutic drugs, such as glycopyrronium bromide, proglumide, cetraxate hydrochloride, cimetidine, and spizofurone; cholagogues, such as ursodesoxycholic acid and osalmid; urogenital and anus agents, such as hexamine, sparteine, dinoprost, ritodrine hydrochloride, oxybutynin, tolterodine, solifenacin, and darifenacin; drugs for parasitic skin diseases, such as salicylic acid, ciclopirox olamine, and coroconazole hydrochloride; skin softeners such as urea; vitamins, such as calcitriol, thiamine hydrochloride, riboflapin sodium phosphate, pyridoxine hydrochloride, nicotinamide, panthenol, and ascorbic acid; mineral preparations, such as calcium chloride, potassium iodide, and sodium iodide; hemostatic drugs such as ethamsylate agents; drugs for liver diseases, such as tiopronin; drugs for habitual intoxication such as cyanamide; therapeutic drugs for gout, such as colchicine, probenecid, and sulfinpyrazone; diabetic drugs, such as tolbutamide, chlorpropamide, glymidine sodium, glypuzole, puformin hydrochloride, and insulin; antibiotics, such as benzylpenicillin potassium, propicillin potassium, cloxacillin sodium, ampicillin sodium, bacampillicin hydrochloride, carbenicillin sodium, cephaloridine, cefoxitin sodium, erythromycin, chloramphenicol, tetracycline, kanamycin sulfate, cycloserine, and metronidazole; chemotherapeutic drugs, such as isocyanide, pyrazinamide, and ethionamide; and narcotics, such as morphine hydrochloride, codeine phosphate, cocaine hydrochloride, pethidine hydrochloride, and fentanyl citrate, antialcoholic drugs such as nalmefene, and anti-obesity drugs such as lorcaserin. Moreover, the salt is not limited to that described above, and various types of salts or free salts can also be used. In the present invention, among the above-described drugs, for example, lidocaine, diclofenac sodium, or tetracaine may be used. Furthermore, in the present invention, among the above-described drugs, drugs that are problematic in terms of solubility, such as donepezil, rotigotine, aripiprazole, and baclofen, may also be used.

The content of the drug or a pharmaceutically acceptable salt thereof in the adhesive layer is not particularly limited. It is generally 0.1% by weight or more and 60% by weight or less, preferably 0.5% by weight or more and 50% by weight or less, more preferably 1% by weight or more and 40% by weight or less, even more preferably 1% by weight or more and 30% by weight or less, and further preferably 2% by weight or more and 30% by weight or less.

### [Other components]

From the viewpoint of enhancing the dispersibility of the drug in the adhesive layer or the transdermal absorbability thereof, the adhesive sheet for skin patch of the present invention may further comprise (e1) an alcohol solvent, (e2) an amide solvent, (e3) an ester solvent, (e4) a liquid organic acid, (e5) a carboxylate, (e6) a lactone, (e7) a surfactant, (e8) an antioxidant, and (e9) a filler, as necessary.

### (e1) Alcohol solvent

Examples of the alcohol solvent may include: higher saturated aliphatic alcohol containing 12 or more and 20 or less carbon atoms that is a liquid at ordinary temperature, such as lauryl alcohol and isostearyl alcohol; higher unsaturated aliphatic alcohol containing 12 or more and 20 or less carbon atoms that is a liquid at ordinary temperature, such as oleyl alcohol; polyhydric alcohol that is a liquid at ordinary temperature, such as ethylene glycol, propylene glycol, glycerin, 1,3-butanediol, and polyethylene glycol having a molecular weight of 100 or more and 600 or less; and propylene glycol mono-fatty acid esters, such as propylene glycol monocaproate, propylene glycol monocaprylate, propylene glycol monocaprate, propylene glycol monolaurate, propylene glycol monomyristate, and propylene glycol monostearate. Among others, from the viewpoint of improving the solubility of a drug, polyhydric alcohol that is a liquid at ordinary temperature, such as ethylene glycol, propylene glycol, glycerin, 1,3-butanediol, propylene glycol monocaprylate, or polyethylene glycol, is preferable; and diol that is a liquid at ordinary temperature, such as ethylene glycol, propylene glycol, 1,3-butanediol, or polyethylene glycol having a molecular weight of 100 or more and 600 or less, propylene glycol monocaprylate is more preferable.

If propylene glycol mono-fatty acid ester is used by being mixed with the present adhesive sheet, drug solubility can be improved without impairing the plasticizing effect of the long-chain branched alcohol. The content of the alcohol solvent such as propylene glycol mono-fatty acid ester is preferably 40 parts by weight or less, more preferably 35 parts by weight or less, further preferably 30 parts by weight or less, and particularly preferably 25 parts by weight or less, with respect to 100 parts by weight of the long-chain branched alcohol. By setting the content of the alcohol solvent such as propylene glycol mono-fatty acid ester to be 40 parts by weight or less with respect to 100 parts by weight of the long-chain branched alcohol, a reduction in the cohesive force of the adhesive base can be prevented.

### (e2) Amide solvent

Examples of the amide solvent may include: pyrrolidone, such as N-methyl-2-pyrrolidone, laurylpyrrolidone, and 2-pyrrolidone; imidazolidinone such as 1,3-dimethyl-2-imidazolidinone; N-substituted toluidine such as crotamiton; and alkanamide, such as formamide, N-methylformamide, N,N-dimethylformamide, N-methylacetamide, N,N-dimethylacetamide, and N-methylpropanamide.

Among the above-described amide solvents, from the viewpoint of improving the solubility, dispersibility and transdermal absorbability of the drug, N-methyl-2-pyrrolidone, laurylpyrrolidone, crotamiton, N,N-dimethylformamide, and N,N-dimethylacetamide are preferable; and N-methyl-2-pyrrolidone and crotamiton are more preferable.

### (e3) Ester solvent

Examples of the ester solvent may include an ester of long-chain fatty acid and monovalent aliphatic alcohol, medium-chain fatty acid triglyceride, an ester of polycarboxylic acid and monovalent aliphatic alcohol, and a carbonate ester.

The ester of long-chain fatty acid and monovalent aliphatic alcohol is preferably an ester of long-chain saturated fatty acid containing 12 or more and 20 or less carbon atoms and monovalent aliphatic alcohol containing 1 or more and 20 or less carbon atoms, wherein the ester is a liquid at ordinary temperature. Examples of such an ester may include: a myristic acid ester that is a liquid at ordinary temperature, such as ethyl myristate, isopropyl myristate, and octyldodecyl myristate; a palmitic acid ester that is a liquid at ordinary temperature, such as ethyl palmitate, isopropyl palmitate, and isostearyl palmitate; and a stearic acid ester that is a liquid at ordinary temperature, such as isopropyl stearate. Also, an ester of long-chain unsaturated fatty acid containing 12 or more and 20 or less carbon atoms and monovalent aliphatic alcohol containing 1 or more and 20 or less carbon atoms can be preferably used. Examples of such an ester may include: an oleic acid ester that is a liquid at ordinary temperature, such as ethyl oleate, decyl oleate, and oleyl oleate; and a linolic acid ester that is a liquid at ordinary temperature, such as ethyl linoleate and isopropyl linoleate.

The medium-chain fatty acid triglyceride is a triglyceride consisting of fatty acid containing 6 or more and 12 or less carbon atoms, such as caproic acid, caprylic acid, capric acid or lauric acid, and glycerin. In the present invention, caprylic acid triglyceride that is a liquid at ordinary temperature, a triglyceride mixture of caprylic acid and capric acid, a triglyceride mixture of caprylic acid, capric acid and lauric acid, etc. can be used. In addition, oil and fat comprising many of these substances that are in a liquid state at ordinary temperature can also be used. Examples of such oil and fat may include peanut oil, olive oil, and castor oil.

Besides, in the present invention, as medium-chain fatty acid triglyceride that is a liquid at ordinary temperature, or as oil and fat comprising the medium-chain fatty acid triglyceride that is a liquid at ordinary temperature, commercially available preparations for pharmaceutical products can also be used.

Examples of the ester of polycarboxylic acid and monovalent aliphatic alcohol may include a diester of dicarboxylic acid containing 2 or more and 12 or less carbon atoms and monovalent aliphatic alcohol containing 1 or more and 20 or less carbon atoms that is a liquid at ordinary temperature, including: an adipic acid diester that is a liquid at ordinary temperature, such as diethyl adipate and diisopropyl adipate; and a sebacic acid diester that is a liquid at ordinary temperature, such as diethyl sebacate, diisopropyl sebacate, and dioctyldecyl sebacate.

The carbonate ester is a cyclic carbonate ester of carbonic acid and diol containing 2 or more and 10 or less carbon atoms, and examples thereof may include ethylene carbonate, propylene carbonate, and vinylene carbonate. Among these, propylene carbonate is preferable.

Among the above-described ester solvents, a myristic acid ester, a medium-chain fatty acid-triglyceride mixture, a sebacic acid diester, and a carbonate ester are preferable; and isopropyl myristate, a triglyceride mixture of caprylic acid and capric acid, diethyl sebacate, propylene carbonate are more preferable.

In the present invention, with regard to the above-described alcohol solvents, amide solvents and ester solvents, one or two or more types can be selected from these solvents and can be used, as necessary. The content of these solvents (i.e., the content of each solvent) is preferably 0.1% by weight or more and 20% by weight or less, and more preferably 0.5% by weight or more and 15% by weight or less, with respect to the total amount of the adhesive layer.

### (e4) Liquid organic acid

Examples of the liquid organic acid may include: aliphatic monocarboxylic acids, such as acetic acid, propionic acid, butyric acid, valeric acid, isovaleric acid, caproic acid, enanthic acid (heptanoic acid), caprylic acid, and pelargonic acid (nonanoic acid); aliphatic unsaturated monocarboxylic acids, such as oleic acid, linoleic acid, arachidonic acid, and docosahexaenoic acid; hydroxycarboxylic acids such as lactic acid (DL-lactic acid, or a mixture of L-lactic acid and/or D-lactic acid and lactic anhydride); liquid carboxylic acids substituted with alkoxy groups, such as methoxyacetic acid; and sulfonic acids such as methanesulfonic acid.

These liquid organic acids have the function of assisting dissolution of a basic drug. These liquid organic acids are able to allow the adhesive layer to comprise a basic drug in a high concentration, are also able to improve the dispersibility of the drug, and further have the effect of improving the transdermal absorbability of the drug. From such viewpoints, among these liquid organic acids, the lactic acid, oleic acid and isostearic acid of Japanese Pharmacopoeia are preferably used, and the lactic acid of Japanese Pharmacopoeia is particularly preferably used.

In the present invention, one or two or more types can be selected from the above-described liquid organic acids and can be added to the adhesive layer, as necessary. The content of the liquid organic acid(s) is preferably 0.1% by weight or more and 20% by weight or less, and more preferably 0.5% by weight or more and 15% by weight or less, with respect to the total amount of the adhesive layer.

### (e5) Carboxylate

Examples of the carboxylate may include the salts of aliphatic monocarboxylic acid, alicyclic monocarboxylic acid, aliphatic dicarboxylic acid, and the like.

Examples of the aliphatic monocarboxylic acid may include: short-chain fatty acids containing 2 to 7 carbon atoms, such as acetic acid, butyric acid, and hexanoic acid; medium-chain fatty acids containing 8 or more and 11 or less carbon atoms, such as octanoic acid and decanoic acid; long-chain fatty acids containing 12 or more carbon atoms, such as myristic acid, stearic acid, isostearic acid, and oleic acid; hydroxy monocarboxylic acids, such as glycolic acid, lactic acid, 3-hydroxybutylic acid, and mandelic acid; monocarboxylic acids substituted with alkoxy groups, such as methoxyacetic acid; and keto monocarboxylic acids such as levulinic acid.

Examples of the alicyclic monocarboxylic acid may include alicyclic monocarboxylic acids containing 6 to 8 carbon atoms, such as cyclohexanecarboxylic acid.

Examples of the aliphatic dicarboxylic acid may include sebacic acid, adipic acid, malic acid, maleic acid, and fumaric acid.

Preferred examples of the carboxylic acid may include long-chain fatty acid containing 12 or more carbon atoms and hydroxy monocarboxylic acid, and specific examples may include myristic acid, stearic acid, isostearic acid, oleic acid, and lactic acid. Among these, oleic acid and lactic acid are more preferable.

Examples of the salts of the above-describe carboxylic acids may include: alkali metal salts, such as sodium salts and potassium salts; alkaline-earth metal salts, such as calcium salts; and amine salts. From the viewpoint of availability and the effect of improving stability and transdermal absorbability, sodium salts are preferably used.

### (e6) Lactone

Examples of the lactone may include 5-membered ring lactones, such as ascorbic acid and isoascorbic acid.

In the adhesive sheet for skin patch of the present invention, taking into consideration the effect of improving the stability of a drug or the effect of improving transdermal absorbability thereof, sodium oleate, sodium lactate, ascorbic acid or isoascorbic acid is preferably used as carboxylate or lactone.

When such carboxylate or lactone is added to the adhesive sheet for skin patch of the present invention, the content of the carboxylate or lactone (i.e., the content of each substance) in the adhesive layer is not particularly limited, and it is preferably 0.1 mole or more and 5 moles or less, and more preferably 0.2 moles or more and 3 moles or less, with respect to 1 mole of the drug. When the additive amount of the carboxylate or lactone is smaller than 0.1 mole with respect to 1 mole of the drug, the effect of improving transdermal absorbability may not be sufficiently obtained. On the other hand, when the additive amount is larger than 5 moles with respect to 1 mole of the drug, the physical properties of the preparation, such as adhesive property, may be deteriorated.

### (e7) Surfactant

Examples of the surfactant may include: polyoxyethylene fatty acid esters, such as polyoxyethylene monolaurate; polyoxyethylene sorbit fatty acid esters, such as polyoxyethylene sorbit tetraoleate; polyoxyethylene sorbitan fatty acid esters, such as polyoxyethylene sorbitan monooleate, polyoxyethylene sorbitan monolaurate, and polyoxyethylene sorbitan monopalmitate; sorbitan fatty acid esters, such as sorbitan monolaurate, sorbitan monooleate, sorbitan sesquioleate, and sorbitan trioleate; glycerin fatty acid esters, such as glycerin monooleate, a polyoxyethylene castor oil derivative, and polyoxyethylene hydrogenated castor oil; polyoxyethylene higher aliphatic alcohol ethers, such as polyoxyethylene lauryl ether and polyoxyethylene oleyl ether; polyoxyethylene alkyl phenyl ethers, such as polyoxyethylene nonyl phenyl ether; polyoxyethylene alkyl amino ethers, such as polyoxyethylene laurylamine and polyoxyethylene oleylamine; nonionic surfactants including polyoxyethylene-polyoxypropylene copolymers, such as Pluronic L-31 and Pluronic L-44; anionic surfactants including alkyl sodium sulfate such as sodium lauryl sulfate; cationic surfactants, such as alkyl trimethyl ammonium salt and alkyl dimethyl ammonium salt; and amphoteric surfactants such as alkyl dimethylamine oxide and alkyl carboxy betaine. One or two or more types can be selected from these surfactants and can be then used.

Among the above-described surfactants, in order to enhance transdermal absorbability, nonionic surfactants that are in a liquid state at ordinary temperature are preferable, sorbitan fatty acid esters that are in a liquid state at ordinary temperature are more preferable, and sorbitan monolaurate is particularly preferable.

When the adhesive sheet for skin patch of the present invention comprises a surfactant, the content of the surfactant in the adhesive layer is preferably 0.01% by weight or more and 10% by weight or less, and more preferably 0.1% by weight or more and 5% by weight or less.

### (e8) Antioxidant

Examples of the antioxidant may include dibutylhydroxytoluene, 4-dioxyphenol, tocopherol, a tocopherol ester derivative, EDTA-2Na, rutin, ascorbic acid, N,N-dimethyl thiourea, L-cysteine, 1-thioglycerol, and 2-mercaptobenzimidazole. Among these, dibutylhydroxytoluene is preferable. The antioxidants may be used alone as a single type, or in combination of two or more types.

The content of the antioxidant is not particularly limited, and the antioxidant can be used in a range in which high skin permeability and the cohesive force and adhesive force sufficient for a patch can be maintained. Among others, the content of the antioxidant is preferably 10% by weight or less, more preferably 5% by weight or less, and most preferably 2% by weight or less, with respect to the total amount of adhesive components.

### (e9) Filler

In order to control the flexibility of the adhesive layer, the adhesive layer may comprise a filler.

Examples of the filler may include: silicon compounds, such as silicic anhydride, light anhydrous silicic acid, and hydrous silicic acid; cellulose derivatives, such as ethyl cellulose, methyl cellulose, hydroxypropyl cellulose, and hydroxypropylmethyl cellulose; water-soluble polymers such as polyvinyl alcohol; aluminum compounds, such as dried aluminum hydroxide gel and hydrous aluminum silicate; and kaoline and titanium oxide. The filler may be used alone or in combination of two or more types.

The content of the filler is not particularly limited, and the filler can be used in a range in which high skin permeability and the cohesive force and adhesive force sufficient for a patch can be maintained. Among others, the content of the filler is preferably 10% by weight or less, more preferably 5% by weight or less, and most preferably 2% by weight or less, with respect to the total amount of adhesive components.

### < Configuration of adhesive sheet for skin patch>

The adhesive sheet for skin patch of the present invention is configured to spread the adhesive layer having the above-described configuration on a backing film.

In the present invention, the "backing film" is not particularly limited, and a backing film commonly used in adhesive sheets for skin patch or transdermal absorption preparations can be used. Examples of such a backing filmmay include: stretchable or non-stretchable woven or non-woven fabrics, such as polyethylene, polypropylene, and polyethylene terephthalate; polyesters, such as polyethylene terephthalate; polyolefins, such as polyethylene and polypropylene; films, such as polyurethane, an ethylene-vinyl acetate copolymer, and vinyl polychloride; and foaming supports, such as polyolefin and polyurethane. These backing films may be used alone, or may also be used as a backing film formed by laminating multiple types of backing films on one another. Moreover, in order to prevent accumulation of static electricity in the backing film, an antistatic agent may be added to the above-described woven fabric, non-woven fabric, film, etc. constituting the backing film. Furthermore, in order to obtain favorable anchoring property with the adhesive layer, a non-woven or woven fabric, or a laminate of such a non-woven or woven fabric and a film can be used. When the backing film is a film, the thickness thereof is generally 10 µm or more and 100 µm or less, and preferably 15 µm or more and 50 µm or less. When the support is a porous sheet such as a woven fabric, a non-woven fabric or a foaming support, the thickness thereof is generally 50 µm or more and 2,000 µm or less, and preferably 100 µm or more and 1,000 µm or less.

Moreover, the adhesive sheet for skin patch of the present invention can also comprise a release liner that is common in the present technical field. Examples of the release liner that can be used herein may include: a glassine paper; polyolefin, such as polyethylene and polypropylene; polyester such as polyethylene terephthalate; a resin film such as polystyrene, an aluminum film, a foaming polyethylene film or a foaming polypropylene film; and a laminate consisting of two or more types selected from the above-described substances. Moreover, these release liners, which are processed with silicone or a fluorine resin, and the release liners subjected to emboss finishing, hydrophilic treatment, hydrophobic treatment, etc., can also be used. The thickness of the release liner is generally 10 µm or more and 200 µm or less, and preferably 15 µm or more and 150 µm or less.

### < Production of adhesive sheet for skin patch>

The adhesive sheet for skin patch of the present invention can be produced, for example, by dissolving or dispersing each of (a) a thermoplastic elastomer, (b) a long-chain branched alcohol, (c) a tackifier, and (d) a drug or a pharmaceutically acceptable salt thereof, in a solvent such as toluene, to prepare a coating solution for the formation of an adhesive layer, then applying the obtained coating solution onto a backing film, and then drying it. In the case of using a release liner, the release liner is crimped to the adhesive layer, so that the release liner can be laminated thereon. Otherwise, the above-described coating solution may be applied onto a release liner, and may be then dried to form an adhesive layer on the surface of the release liner, and thereafter, a backing film may be crimped onto the adhesive layer, so that they may adhere to each other.

As a solvent used for the coating solution, a solvent capable of uniformly dissolving or dispersing therein the above (a), the above (b), the above (c), and the above (d) is preferable. Examples of such a solvent may include: aromatic hydrocarbons such as toluene; alicyclic hydrocarbons, such as cyclohexane and methylcyclohexane; aliphatic hydrocarbons, such as hexane and heptane; ethers, such as tetrahydrofuran, diethyl ether, and t-butylmethyl ether; ketones, such as acetone, methyl ethyl ketone, and methyl isobutyl ketone; alcohols, such as ethanol, propanol, and butanol; and acetic acid esters, such as ethyl acetate, propyl acetate, isopropyl acetate, butyl acetate, and isobutyl acetate. These solvents can be used alone or in combination of two or more types. Because of favorable solubility of individual components constituting the adhesive layer, it is more preferable to use aromatic hydrocarbons such as toluene, alicyclic hydrocarbons, such as cyclohexane and methylcyclohexane, and aliphatic hydrocarbons, such as hexane and heptane, alone or in combination; or it is more preferable to combine aromatic hydrocarbons such as toluene, or aliphatic hydrocarbons, such as hexane and heptane, with acetic acid esters, such as ethyl acetate, propyl acetate, isopropyl acetate, butyl acetate, and isobutyl acetate, and thus, to use the obtained mixture as a solvent.

The coating solution used for the formation of the adhesive layer can be applied using a commonly used coater, such as, for example, a roll coater, a die coater, a gravure coater, a reverse roll coater, a kiss-roll coater, a dip roll coater, a bar coater, a knife coater, or a spray coater. Moreover, the coating solution is preferably dried under heating, for example, at a temperature of 40°C or higher and 150°C or lower. The drying temperature, the drying time, and the drying system may be adjusted depending on the used solvent or the used amount. The weight per unit area of the dried adhesive layer may be adjusted depending on necessary skin adhesiveness or transdermal absorbability. The weight per unit area of the dried adhesive layer, which achieves skin adhesiveness and is within a producible range, is preferably 10 g/m² or more and 1,000 g/m² or less, more preferably 20 g/m² or more and 800 g/m² or less, and further preferably 30 g/m² or more and 600 g/m² or less.

The present application claims priority from Japanese Patent Application No. 2018-008106, filed on January 22, 2018; the disclosure of which is incorporated herein by reference in their entirety.

### Examples

Hereinafter, the present invention will be more specifically described in the following examples and comparative examples. However, these examples are not intended to limit the scope of the present invention. The numerical value shown in the tables in the examples indicates part by weight.

### (Evaluation Method) Cohesive force

The cohesive force of a patch was evaluated based on the following viewpoints.
○: Adhesive residue was not found.
Δ: Cohesive force was slightly insufficient, but it did not cause problem.
×: Adhesive residue, deformation, etc. were found, and the lack of cohesive force was significant.

### (Evaluation Method) Adhesiveness

The adhesiveness of a patch was evaluated based on the following viewpoints.
○: The present patch exhibited higher adhesiveness than the existing local anesthetic patch (lidocaine tape) comprising a rubber-based adhesive base.
Δ: The present patch exhibited slightly lower adhesiveness than the existing local anesthetic patch (lidocaine tape) comprising a rubber-based adhesive base.
×: Significant peeling was found.

### (Evaluation Method) Evaluation of skin permeability

The abdominal extraction skin of a depilated male Wister rat (5 weeks old) was attached to a vertical Franz diffusion cell (manufactured by Vidrex Co. Ltd., model: TP-8s). The patches produced in Examples and Comparative Examples were each cut into a round shape with a dimeter of 1.3 cm, and thereafter, they were each attached onto the rat skin in the Franz diffusion cell. As a buffer, 5% ethanol-containing 0.01 mol/L phosphate buffered saline (pH 7.2 to 7.4) was used, and the test was performed at a buffer temperature of 32°C. After initiation of the test, a moiety of the buffer was sampled at regular intervals, and the amount of lidocaine permeating through the rat skin in the buffer was quantified according to HPLC.

### [Examples 1 to 5 and Comparative Examples 1 to 6]

### Preparation of adhesive sheets for skin patch

In accordance with the prescriptions shown in Tables 1 and 2, individual components constituting an adhesive layer were weighed. First, a styrene-isoprene-styrene block copolymer (manufactured by ZEON CORPORATION, "Quintac (registered trademark) 3520") was dissolved in toluene. Thereafter, in Examples 1 to 5 and Comparative Examples 1 and 2, 2-hexyl-1-decanol was added to the solution, in Comparative Examples 3 and 4, lauryl alcohol was added to the solution, and in Comparative Examples 5 and 6, oleyl alcohol was added to the solution, followed by blending and stirring the mixed solution, so as to prepare each coating solution for the formation of an adhesive layer.

The above-described coating solution was applied onto a polyethylene terephthalate (PET)-made film (release liner) that had been treated with silicone, so as to prepare an adhesive layer having a thickness after drying of approximately 400 µm. The adhesive layer was dried in an oven at 50°C for 60 minutes, and a PET-made film (backing film) was then laminated on the surface of the adhesive layer. The resultant was cut into a size of 15 cm x 30 cm, thereby obtaining a desired adhesive sheet for skin patch.

**[Table 1]**

| | Ex. 1 | Ex. 2 | Ex. 3 | Ex. 4 | Ex. 5 |
|---|---|---|---|---|---|
| 2-Hexyl-1-decanol | 66.7 | 60.0 | 50.0 | 42.9 | 33.3 |
| Styrene-isoprene-styrene copolymer, Quintac 3520 | 33.3 | 40.0 | 50.0 | 57.1 | 66.7 |
| Cohesive force | ○ | ○ | ○ | ○ | ○ |
| Adhesive force | ○ | ○ | ○ | ○ | Δ |

**[Table 2]**

| | Comp. Ex. 1 | Comp. Ex. 2 | Comp. Ex. 3 | Comp. Ex. 4 | Comp. Ex. 5 | Comp. Ex. 6 |
|---|---|---|---|---|---|---|
| 2-Hexyl-1-decanol | 71.4 | 20.0 | - | - | - | - |
| Lauryl alcohol | - | - | 66.7 | 33.3 | - | - |
| Oleyl alcohol | - | - | - | - | 66.7 | 33.3 |
| Styrene -isoprene- styrene copolymer, Quintac 3520 | 28.6 | 80.0 | 33.3 | 66.7 | 33.3 | 66.7 |
| Cohesive force | × | ○ | ○* | ○ | ○* | ○ |
| Adhesive force | - | × | - | × | - | × |

| | | | | | | |
|---|---|---|---|---|---|---|
| * Bleeding was generated. | | | | | | |

All of the adhesive sheets for skin patch of Examples 1 to 5 exhibited favorable cohesive force and adhesive force. On the other hand, with regard to Comparative Example 1, in accordance with the prescription of Table 2, individual components constituting the adhesive layer were weighed, and a patch was prepared by the above-described method. However, the adhesive sheet for skin patch of Comparative Example 1 had too low cohesive force and could not maintain the adhesive layer, and thus, adhesive force could not be evaluated. With regard to Comparative Example 2, adhesive force was low, and thus, the adhesive sheet was peeled from the skin immediately after it had been attached thereto. With regard to Comparative Examples 3 and 5, the peeling (delamination) of the support was observed, and the oozing out (bleeding) of the liquid component was generated, and thus, adhesive force could not be evaluated. In addition, Comparative Examples 4 and 6, adhesive force was low, and thus, the adhesive sheet was peeled from the skin immediately after it had been attached thereto. Further, delamination was also observed.

### [Examples 6 to 10 and Comparative Examples 7 to 9]

### Preparation of patches

In accordance with the prescriptions shown in Tables 3 and 4, individual components constituting an adhesive layer were weighed. First, a styrene-isoprene-styrene block copolymer ("Quintac (registered trademark) 3520" manufactured by ZEON CORPORATION; and "5505" manufactured by JSR) was dissolved in toluene. Thereafter, 2-hexyl-1-decanol and 2-octyl-1-dodecanol was added to the solution, and lidocaine was further added thereto, followed by blending and stirring the obtained mixture, so as to prepare a coating solution for the formation of an adhesive layer.

The above-described coating solution was applied onto a polyethylene terephthalate (PET)-made film (release liner) that had been treated with silicone, so as to prepare an adhesive layer having a thickness after drying of approximately 400 µm. The adhesive layer was dried in an oven at 50°C for 60 minutes, and a PET-made film (backing film) was then laminated on the surface of the adhesive layer. The resultant was cut into a size of 15 cm x 30 cm, thereby obtaining a desired patch.

**[Table 3]**

| | Ex. 6 | Ex. 7 | Ex. 8 | Ex. 9 | Ex. 10 |
|---|---|---|---|---|---|
| Lidocaine | 30.0 | 30.0 | 30.0 | 30.0 | 30.0 |
| 2-Hexyl-1-decanol | 35.0 | - | 46.7 | 42.0 | 23.3 |
| 2-Octyl-1-dodecanol | - | 35.0 | - | - | - |
| Styrene-isoprene-styrene copolymer, Quintac 3520/5505 | 35.0 | 35.0 | 23.3 | 28.0 | 46.7 |
| Cohesive force | ○ | ○ | Δ - ○ | ○ | ○ |
| Adhesive force | ○ | ○ | ○ | ○ | Δ - ○ |

**[Table 4]**

| | Comp. Ex. 7 | Comp. Ex. 8 | Comp. Ex. 9 |
|---|---|---|---|
| Lidocaine | 30.0 | 30.0 | 30.0 |
| 2-Butyl-1-octanol | 35.0 | - | - |
| Lauryl alcohol | - | 35.0 | - |
| Oleyl alcohol | - | - | 35.0 |
| Styrene-isoprene-styrene copolymer, Quintac 3520/5505 | 35.0 | 35.0 | 35.0 |
| Cohesive force | × | ○* | × |
| Adhesive force | - | - | - |

| | | | |
|---|---|---|---|
| * Bleeding was generated. | | | |

All of the patches of Examples 6 to 10 exhibited favorable cohesive force and adhesive force. On the other hand, with regard to Comparative Example 7 using a long-chain branched alcohol having a short carbon chain and Comparative Example 9 using a linear oleyl alcohol, in accordance with the prescriptions of Table 4, individual components constituting the adhesive layer were weighed, and a patch was prepared by the above-described method. However, the patches of Comparative Examples 7 and 9 had too low cohesive force and could not maintain the adhesive layer, and thus, adhesive force could not be evaluated. With regard to Comparative Example 8, the peeling (delamination) of the support was observed, and the oozing out (bleeding) of the liquid component was generated, and thus, adhesive force could not be evaluated.

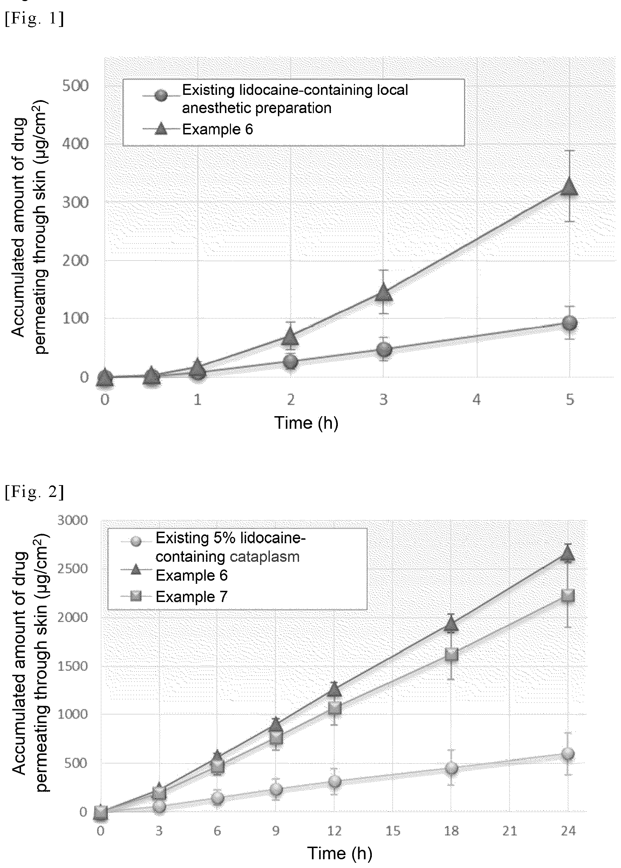

Subsequently, an existing lidocaine-containing local anesthetic preparation comprising a rubber-based adhesive base (product name: Yupatch (registered trademark) (Yutoku Pharmaceutical IND. Co., Ltd.)) was used as a control preparation, and the patch of Example 6 was measured in terms of skin permeability. The results are shown in Fig. 1. Five hours after initiation of the test, the accumulated amount of the drug permeating through the skin was exhibited to be a high value, which was 3.5 times larger than that of the existing local anesthetic preparation.

In addition, a skin permeability test was carried out, also using a 5% lidocaine-containing hydrogel patch (product name: LIDODERM (registered trademark) (Teikoku Seiyaku Co., Ltd.)), which had been placed on the market as a postherpetic neuralgia treatment agent in U.S.A., as a control preparation. The results are shown in Fig. 2. As shown in Fig. 2, twenty-four hours after initiation of the test, in terms of the accumulated amount of the drug permeating through the skin, the patches of Example 6 and Example 7 exhibited high values, which were 4.5 times and 3.7 times larger than that of the existing 5% lidocaine-containing hydrogel patch, respectively.

In accordance with the prescriptions shown in Table 5, the patches described in Patent Document 2 were prepared (Comparative Examples 10 and 11). A styrene-isoprene-styrene block copolymer ("Quintac (registered trademark) 3520" manufactured by ZEON CORPORATION; and "5002" manufactured by JSR) was used. BHT indicates dibutylhydroxytoluene.

**[Table 5]**

| | Comp. Ex. 10 | Comp. Ex. 11 |
|---|---|---|
| Diclofenac sodium | 10.0 | 10.0 |
| Styrene-isoprene-styrene copolymer, Quintac 3520/5002 | 23.8 | 23.8 |
| Terpene resin | 35.6 | 35.6 |
| BHT | 0.6 | 0.6 |
| Oleyl alcohol | 30.0 | - |
| Lauryl alcohol | - | 30.0 |
| Cohesive force | × | × |
| Adhesive force | ○ | ○ |

In the patches of Comparative Examples 10 and 11, since diclofenac sodium was not dissolved in each linear long-chain alcohol, it was dispersed in the adhesive base. Moreover, the cohesiveness of the adhesive base was low, and thus, bleeding was observed upon the peeling of the liner.

In the patches of Examples 6 to 10, crystal precipitation was not confirmed under conditions of room temperature without artificial control (23°C). On the other hand, under conditions of 4°C, crystal precipitation was confirmed in the patch of Example 10. In the patches of Examples 6 to 9, although crystal precipitation was observed upon the storage under conditions of 4°C, such crystals disappeared when the temperature was returned to room temperature.

**[Table 6]**

| | Ex. 6 | Ex. 7 | Ex. 8 | Ex. 9 | Ex. 10 |
|---|---|---|---|---|---|
| Crystal precipitation at room temperature without artificial control (23°C) | No | No | No | No | No |
| Crystal precipitation at 4°C | No* | No* | No* | No* | Yes |

### [Examples 11 and 12 and Comparative Example 12]

### Preparation of patches

In accordance with the prescriptions shown in Table 7, individual components constituting an adhesive layer were weighed to prepare a coating solution for the formation of an adhesive layer. The above-described coating solution was applied onto a polyethylene terephthalate (PET)-made film (release liner) that had been treated with silicone, so as to prepare an adhesive layer having a thickness after drying of approximately 400 µm. The adhesive layer was dried in an oven at 50°C for 60 minutes, and a PET-made film (backing film) was then laminated on the surface of the adhesive layer. The resultant was cut into a size of 15 cm x 30 cm, thereby obtaining a desired patch.

**[Table 7]**

| | Ex. 11 | Ex. 12 | Comp. Ex. 12 |
|---|---|---|---|
| Tetracaine | 4.0 | 4.0 | 4.0 |
| 2-Hexyl-1-decanol | 23.0 | - | - |
| 2-Octyl-1-dodecanol | - | 23.0 | - |
| Styrene-isoprene-styrene copolymer, Quintac 3520 | 46.0 | 46.0 | 22.0 |
| Octyldodecyl myristate | 17.0 | 17.0 | - |
| Capryol 90 (Gattefosse) | 10.0 | 10.0 | - |
| Liquid paraffin Kaydol | - | - | 74.0 |
| Cohesive force | ○ | ○ | ○ |
| Adhesive force | ○ | ○ | ○ |
| Crystal precipitation at 4°C | No | No | Yes |

The patches of Examples 11 and 12 both exhibited favorable cohesive force and adhesive force. In addition, when crystal precipitation was examined under conditions of the storage at 4°C, such crystal precipitation was not observed. On the other hand, although Comparative Example 12, in which liquid paraffin was used as a plasticizer, exhibited favorable cohesive force and adhesive force, precipitation of crystals was observed upon the storage at 4°C.

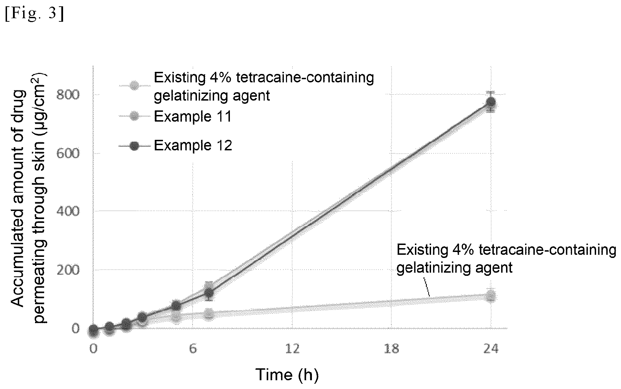

Subsequently, an existing 4% tetracaine-containing gelatinizing agent (product name: Ametop) was used as a control preparation, and the patches of Examples 11 and 12 were measured in terms of skin permeability. The results are shown in Fig. 3. Until 3 hours after initiation of the test, the patches of Examples 11 and 12 exhibited almost the same level of skin permeability as the control preparation, and after 3 hours, the patches exhibited higher skin permeability than the control preparation. Twenty-four hours after initiation of the test, in terms of the accumulated amount of the drug permeating through the skin, the patches of Examples 11 and 12 each exhibited an extremely high value, which was 6.6 times higher than the control preparation.

### [Examples 13 to 15]

### Preparation of patches

In accordance with the prescriptions shown in Table 8, individual components constituting an adhesive layer were weighed to prepare a coating solution for the formation of an adhesive layer. The above-described coating solution was applied onto a polyethylene terephthalate (PET)-made film (release liner) that had been treated with silicone, so as to prepare an adhesive layer having a thickness after drying of approximately 400 µm. The adhesive layer was dried in an oven at 50°C for 60 minutes, and a PET-made film (backing film) was then laminated on the surface of the adhesive layer. The resultant was cut into a size of 15 cm x 30 cm, thereby obtaining a desired patch.

All of the patches of Examples 13 to 15 exhibited favorable cohesive force and adhesive force.

**[Table 8]**

| | Ex. 13 | Ex. 14 | Ex. 15 |
|---|---|---|---|
| Lidocaine | 30 | 30 | 30 |
| Styrene-isoprene-styrene copolymer Quintac 3520 | 35 | 35 | 35 |
| 2-Octyl-1-dodecanol | 25 | 25 | 25 |
| Terpene resin (tackifier) PX1150N (YASUHARA CHEMICAL CO., | 10 | | |
| LTD.) | | | |
| Alicyclic saturated hydrocarbon resin (tackifier) Aroconp100 (Arakawa Chemical Industries, Ltd.) | | 10 | |
| Rosin (tackifier) KE-311 (Arakawa Chemical Industries, Ltd.) | | | 10 |
| Cohesive force | ○ | ○ | ○ |
| Adhesive force | ○ | ○ | ○ |

### [Examples 16 to 22 and Comparative Examples 13 to 19]

### Preparation of patches

In accordance with the prescriptions shown in Table 9 and Table 10, individual components constituting an adhesive layer were weighed to prepare a coating solution for the formation of an adhesive layer. The above-described coating solution was applied onto a polyethylene terephthalate (PET)-made film (release liner) that had been treated with silicone, so as to prepare an adhesive layer having a thickness after drying of approximately 400 µm. The adhesive layer was dried in an oven at 50°C for 60 minutes, and a PET-made film (backing film) was then laminated on the surface of the adhesive layer. The resultant was cut into a size of 15 cm x 30 cm, thereby obtaining a desired patch.

**[Table 9]**

| | Ex. 16 | Ex. 17 | Ex. 18 | Ex. 19 | Ex. 20 | Ex. 21 | Ex. 22 |
|---|---|---|---|---|---|---|---|
| Lidocaine | | | | | | | |
| Tetracaine | 4.0 | | | | | | |
| Donepezil | | 4.0 | | | | | |
| Rotigotine | | | 6.0 | | | | |
| Ketoprofen | | | | 7.0 | | | |
| Clonidine | | | | | 3.0 | | |
| Ibuprofen | | | | | | 15.0 | |
| Diclofenac epolamine salt | | | | | | | 5.0 |
| Styrene-isoprene-styrene copolymer Quintac 3520 | 35.0 | 35.0 | 35.0 | 35.0 | 35.0 | 35.0 | 35.0 |
| 2-Hexyl-1-decanol | 61.0 | 61.0 | 59.0 | 58.0 | 62.0 | 50.0 | 60.0 |
| Liquid paraffin Kaydol | | | | | | | |
| Cohesive force | ○ | ○ | ○ | ○ | ○ | ○ | ○ |
| Adhesive force | ○ | ○ | ○ | ○ | ○ | ○ - Δ | ○ |
| Crystal precipitation at 4°C | No | No | No | No | No | No | No |

**[Table 10]**

| | Comp. Ex. 13 | Comp. Ex. 14 | Comp. Ex. 15 | Comp. Ex. 16 | Comp. Ex. 17 | Comp. Ex. 18 | Comp. Ex. 19 |
|---|---|---|---|---|---|---|---|
| Lidocaine | 3.0 | | | | | | |
| Tetracaine | | | | | | | |
| Donepezil | | 1.0 | | | | | |
| Rotigotine | | | 1.0 | | | | |
| Ketoprofen | | | | 0.5 | | | |
| Clonidine | | | | | 0.3 | | |
| Ibuprofen | | | | | | 3.0 | |
| Diclofenac epolamine salt | | | | | | | 0.5 |
| Styrene-isoprene-styrene copolymer Quintac 3520 | 35.0 | 35.0 | 35.0 | 35.0 | 35.0 | 35.0 | 35.0 |
| 2-Hexyl-1-decanol | | | | | | | |
| Liquid paraffin Kaydol | 62.0 | 64.0 | 64.0 | 64.5 | 64.7 | 62.0 | 64.5 |
| Cohesive force | ○ | ○ - Δ | ○ - Δ | ○ | ○ | ○ | ○ |
| Adhesive force | ○ | ○ | ○ | ○ | ○ | ○ | ○ |
| Crystal precipitation at 4°C | Yes | Yes | Yes | Yes | Yes | Yes | Yes |

All of the patches of Examples 16 to 22 exhibited favorable cohesive force and adhesive force. In addition, when crystal precipitation was examined under conditions of the storage at 4°C, such crystal precipitation was not observed. On the other hand, with regard to Comparative Examples 13 to 19, in all of which liquid paraffin was used as a plasticizer, precipitation of crystals was observed upon the storage at 4°C.

### [Examples 23 to 27]

### Preparation of patches

In accordance with the prescriptions shown in Table 11, individual components constituting an adhesive layer were weighed to prepare a coating solution for the formation of an adhesive layer. The above-described coating solution was applied onto a polyethylene terephthalate (PET)-made film (release liner) that had been treated with silicone, so as to prepare an adhesive layer having a thickness after drying of approximately 400 µm. The adhesive layer was dried in an oven at 50°C for 60 minutes, and a PET-made film (backing film) was then laminated on the surface of the adhesive layer. The resultant was cut into a size of 15 cm x 30 cm, thereby obtaining a desired patch.

**[Table 11]**

| | Ex. 23 | Ex. 24 | Ex. 25 | Ex. 26 | Ex. 27 |
|---|---|---|---|---|---|
| Styrene-isoprene-styrene copolymer Quintac 3520 | 64.1 | 41.7 | 26.3 | 25.6 | 25.0 |
| 2-Hexyl-1-decanol | 25.6 | 41.7 | 52.6 | 51.3 | 50.0 |
| Propylene glycol monocaprylate (product name: Capryol PGMC) | 10.3 | 16.7 | 21.1 | 23.1 | 25.0 |
| Cohesive force | ○ | ○ | ○ - Δ | ○ - Δ | O - Δ |
| Adhesive force | Δ | ○ | ○ | Δ | Δ |

Even in a case where propylene glycol monocaprylate that is a propylene glycol mono-fatty acid ester was used as an alcohol solvent, the patches exhibited acceptable cohesive force and adhesive force.

## Claims

1. An adhesive sheet for skin patch, having an adhesive layer on a support, wherein
the adhesive layer comprises, at least, a thermoplastic elastomer and a long-chain branched alcohol having a total carbon number of 13 or more,
the long-chain branched alcohol is comprised in an amount of 40 parts by weight or more and 200 parts by weight or less, with respect to 100 parts by weight of the thermoplastic elastomer, and
the adhesive layer does not comprise a tackifier, or comprises a tackifier in an amount of 30% by weight or less, with respect to the amount of the entire adhesive layer.

2. The adhesive sheet for skin patch according to claim 1, which comprises the long-chain branched alcohol in an amount of 50 parts by weight or more and 200 parts by weight or less, with respect to 100 parts by weight of the thermoplastic elastomer.

3. The adhesive sheet for skin patch according to claim 1 or 2, which comprises the long-chain branched alcohol in an amount of 10% by weight or more, with respect to the amount of the entire adhesive layer.

4. The adhesive sheet for skin patch according to any one of claims 1 to 3, which comprises the long-chain branched alcohol in an amount of 20% by weight or more, with respect to the amount of the entire adhesive layer.

5. The adhesive sheet for skin patch according to any one of claims 1 to 4, wherein the thermoplastic elastomer is a styrene-based block copolymer.

6. The adhesive sheet for skin patch according to claim 5, wherein the styrene-based block copolymer is a mixture consisting of a styrene-isoprene-styrene block copolymer and a styrene-isoprene block copolymer.

7. The adhesive sheet for skin patch according to claim 6, wherein the content of the styrene-isoprene block copolymer in the mixture is 30% by weight or more.

8. The adhesive sheet for skin patch according to claim 6 or 7, wherein the content of the styrene-isoprene block copolymer in the mixture is 50% by weight or more.

9. The adhesive sheet for skin patch according to any one of claims 1 to 8, wherein the content of the tackifier is 20% by weight or less, with respect to the content of the entire adhesive layer.

10. The adhesive sheet for skin patch according to any one of claims 1 to 9, wherein the content of the tackifier is 15% by weight or less, with respect to the content of the entire adhesive layer.

11. The adhesive sheet for skin patch according to any one of claims 1 to 10, wherein the content of the tackifier is 10% by weight or less, with respect to the content of the entire adhesive layer.

12. The adhesive sheet for skin patch according to any one of claims 1 to 11, which does not comprise a tackifier.

13. The adhesive sheet for skin patch according to any one of claims 1 to 12, wherein the adhesive layer further comprises an alcohol solvent in an amount of 40 parts by weight or less, with respect to 100 parts by weight of the long-chain branched alcohol.

14. The adhesive sheet for skin patch according to claim 13, wherein the alcohol solvent is propylene glycol mono-fatty acid ester.

15. An adhesive sheet for skin patch, having an adhesive layer on a support, wherein the adhesive layer comprises, at least, a thermoplastic elastomer and, as a plasticizer, a long-chain branched alcohol having a total carbon number of, at least, 13 or more, and the plasticizer is comprised in an amount of 20% by weight or more, with respect to the amount of the entire adhesive layer.

16. The adhesive sheet for skin patch according to any one of claims 1 to 15, wherein the adhesive layer further comprises a drug or a pharmaceutically acceptable salt thereof.

17. The adhesive sheet for skin patch according to claim 16, which is used as a patch.
